# EUROPEAN PATENT APPLICATION

(11) **EP 2 457 906 A1**
(43) Date of publication of application: **30.05.2012**
(21) Application number: 11191070.9
(22) Date of filing: 29.11.2011
(51) Int. Cl.: C07D 285/24, C07D 417/04, C07D 419/04, A61K 31/5415, A61P 29/00

(54) **Derivatives of 1,2,4-benzothiadiazine dioxide, their preparation and use as allosteric modulators of the AMPA receptor**

(30) Priority: 29.11.2010 IT MI20102215
(71) Applicant: Universita'del Salento, 73100 Lecce (IT); Uniwersytet Medyczny W Lublinie, 20-059 Lublin (PL)
(72) Inventor: Battisti, Umberto, 41100 Modena (IT); Cannazza, Giuseppe, 40135 Bologna (IT); Puja, Giulia, 41100 Modena (IT); Carrozzo, Marina Maria, 73031 Alessano Lecce (IT); Braghiroli, Daniela, 41037 Mirandola (IT); Parenti, Carlo, 40014 Crevalcore (IT); Troisi, Luigino, 73055 Racale (IT); Jozwiak, Krzysztof, 20-819 Lublin (PL)
(74) Representative: Villa, Livia

(57) **Abstract**

A compound of formula I is described: 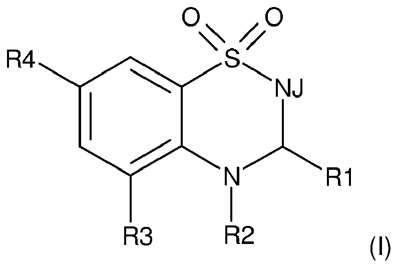 which showed to be surprisingly and significantly effective as an allosteric modulator of the AMPA receptor and is therefore particularly suitable for the treatment of mnemocognitive disorders ascribable to an alteration of glutamatergic neurotransmission, such as age-related disorders, anxiety, progressive neurodegenerative disorders, depressive disorders, schizophrenia, obsessive-compulsive disorders and bipolar disorder.

## Description

### FIELD OF THE INVENTION

The present invention relates to derivatives of 1,2,4-benzothiadiazine 1,1-dioxide, which have been proven to be effective as allosteric modulators of the AMPA receptor, therefore finding advantageous application in the treatment of mnemocognitive disorders ascribable to an alteration of glutamatergic neurotransmission, such as age-related disorders, anxiety, progressive neurodegenerative disorders, depressive disorders, schizophrenia, obsessive-compulsive disorders and bipolar disorder.

### PRIOR ART

L-glutamate is the main excitatory neurotransmitter in the mammalian central nervous system. Pathophysiological studies have clearly demonstrated that a deficit in glutamatergic neurotransmission is closely associated with the development of various neurodegenerative diseases, such as Alzheimer's disease (Neuroscience and Biobehavioral Reviews, 1992, 16, 13-24; Progress in Neurobiology, 1992, 39, 517-545). Ionotropic glutamate receptors are divided into three subtypes according to their selective response to specific agonists: α-amino-3-hydroxy-5-methylisoxazolo-4-propionic acid (AMPA), N-methyl-D-aspartate (NMDA) and kainate. Among these, the AMPA receptor seems to be most involved in the physiological phenomena of neuronal excitation, and in particular in the processes of memory formation. It has in fact been noted that the phenomenon of learning is correlated with an increase in the binding of AMPA its receptor in the hippocampus, one of the most important regions for mnemocognitive processes. When activated by glutamate, the AMPA receptor ion channels allow sodium ions (Na⁺) and calcium ions (Ca⁺⁺) to pass directly through the channel.

The ion channel currents activated by glutamate are transient; in fact, these ion channels enter a refractory state, called desensitisation, in which the glutamate is linked to the receptor.

There is therefore a need to intervene on the AMPA receptor in order to inhibit the desensitisation in cases where it is desirable to potentiate glutamatergic neurotransmission, for example in pathologies such as age-related disorders, anxiety, progressive neurodegenerative disorders, depressive disorders and schizophrenia, or in order to increase the desensitisation, in cases where it is desirable to decrease glutamatergic neurotransmission, for example in pathologies such as obsessive-compulsive and bipolar disorders. The object of the present invention is therefore to meet this need.

### SUMMARY OF THE INVENTION

This object has been achieved by a compound of formula I: 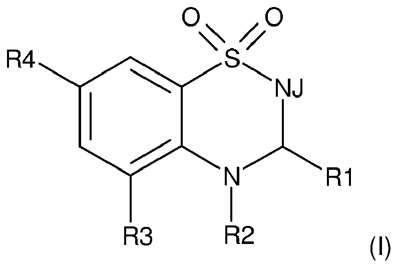 an enantiomer, diastereoisomer, or salt thereof with a pharmaceutically acceptable acid or base, wherein:
J, if present, is H,
R1 and R2 are, independently of each other, H, C₁-C₃ alkyl or C₁-C₃ alkyl substituted with at least one halogen,
R3 is a heterocyclic or aryl group, where said heterocyclic group is monocyclic or bicyclic, aromatic or aliphatic, comprising at least one heteroatom selected from nitrogen, oxygen or sulphur, and said aryl group is monocyclic or bicyclic, having at least one aromatic ring, or is a heterocyclic or aryl group substituted with at least one substituent group selected from C₁-C₃ alkyl, halogen, -OH, -CN, -COOH, -NO₂, -OY1, -COOY1, -NY1Y2, - (SO₂)NY1Y2, where Y1 and Y2 are, independently of each other, H or C₁-C₆ alkyl linear or branched,
R4 is H, C₁-C₆ alkyl linear or branched, halogen, -CN, -NO₂, -OY1, -COOY1, -NY1 Y2, -(SO₂)NY1Y2, where Y1 and Y2 are, independently of each other, H or C₁-C₆ alkyl linear or branched, or R4 is Z1, -OZ1, -OCOZ1 or -OCNZ1, where Z1 is a heterocyclic or aryl group, where said heterocyclic group is monocyclic or bicyclic, aromatic or aliphatic, comprising at least one heteroatom selected from nitrogen, oxygen or sulphur, and said aryl group is monocyclic or bicyclic, having at least one aromatic ring, or is a heterocyclic or aryl group substituted with at least one substituent group selected from C₁-C₃ alkyl, halogen, -OH, -CN, -NY1Y2, -(SO₂)NY1Y2, where Y1 and Y2 are, independently of each other, H or C₁-C₆ alkyl linear or branched.

In another aspect, the present invention relates to a process for the preparation of the compound of formula (I), comprising the steps of:
a) halogenation at position 3 of the compound of formula (i) 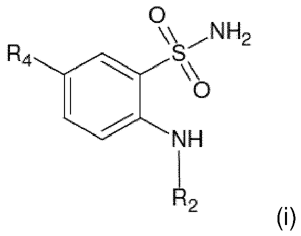
b) condensation by reaction with B(OH)₂R3, and
c) cyclisation by reaction with R1COH.

In a further aspect, the present invention relates to the use of the compound of formula (I) as an allosteric modulator of the AMPA receptor in the treatment of mnemocognitive disorders ascribable to an alteration of glutamatergic neurotransmission, such as age-related disorders, anxiety, progressive neurodegenerative disorders, depressive disorders, schizophrenia, obsessive-compulsive disorders and bipolar disorder.

As will be evident from the following detailed description, it was in fact found that the compound of formula (I) acts as an allosteric modulator of the AMPA receptor, intervening on the synaptic currents induced by glutamate.

### BRIEF DESCRIPTION OF THE FIGURE

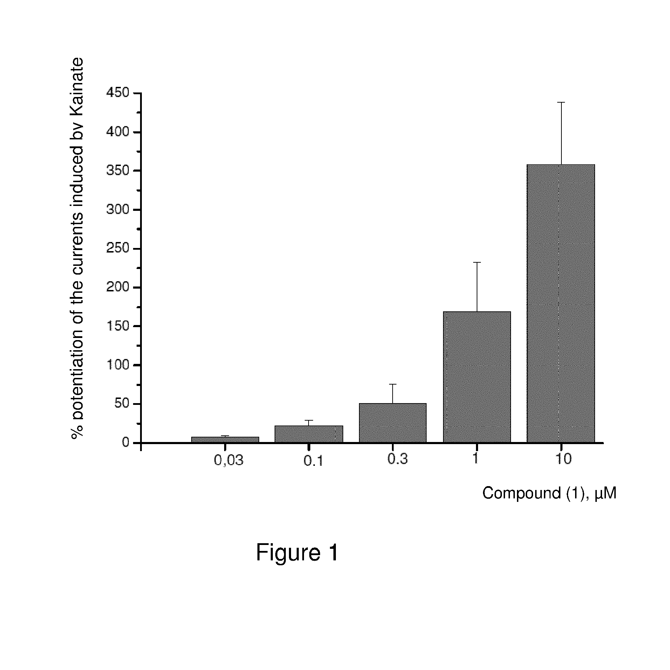

The characteristics and advantages of the present invention will be evident from the following detailed description, from the working examples given for illustrative and non-limiting purposes, and from the annexed Figure 1, which displays a graph of the % potentiation of the currents induced by Kainate in the presence of increasing concentrations of the compound obtained according to Example 1. Each bar represents the mean of the data ± the standard error (SE) from 4-6 cells (rat cortical neurons).

### DETAILED DESCRIPTION OF THE INVENTION

The present invention therefore relates to a compound of formula I: 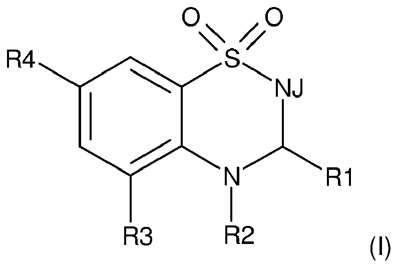 an enantiomer, diastereoisomer, or salt thereof with a pharmaceutically acceptable acid or base, wherein:
J, if present, is H,
R1 and R2 are, independently of each other, H, C₁-C₃ alkyl or C₁-C₃ alkyl substituted with at least one halogen,
R3 is a heterocyclic or aryl group, where said heterocyclic group is monocyclic or bicyclic, aromatic or aliphatic, comprising at least one heteroatom selected from nitrogen, oxygen or sulphur, and said aryl group is monocyclic or bicyclic, having at least one aromatic ring, or is a heterocyclic or aryl group substituted with at least one substituent group selected from C₁-C₃ alkyl, halogen, -OH, -CN, -COOH, -NO₂, -OY1, -COOY1, -NY1Y2, - (SO₂)NY1Y2, where Y1 and Y2 are, independently of each other, H or C₁-C₆ alkyl linear or branched,
R4 is H, C₁-C₆ alkyl linear or branched, halogen, -CN, -NO₂, -OY1, -COOY1, -NY1 Y2, -(SO₂)NY1Y2, where Y1 and Y2 are, independently of each other, H or C₁-C₆ alkyl linear or branched, or R4 is Z1, -OZ1, -OCOZ1 or -OCNZ1, where Z1 is a heterocyclic or aryl group, where said heterocyclic group is monocyclic or bicyclic, aromatic or aliphatic, comprising at least one heteroatom selected from nitrogen, oxygen or sulphur, and said aryl group is monocyclic or bicyclic, having at least one aromatic ring, or is a heterocyclic or aryl group substituted with at least one substituent group selected from C₁-C₃ alkyl, halogen, -OH, -CN, -NY1Y2, -(SO₂)NY1Y2, where Y1 and Y2 are, independently of each other, H or C₁-C₆ alkyl linear or branched.

The pharmaceutically acceptable acids include hydrochloric acid, hydrobromic acid, sulphuric acid, phosphonic acid, acetic acid, trifluoroacetic acid, lactic acid, pyruvic acid, malonic acid, succinic acid, glutaric acid, fumaric acid, tartaric acid, maleic acid, citric acid, ascorbic acid, methanesulfonic acid and camphoric acid. The pharmaceutically acceptable bases include sodium hydroxide, potassium hydroxide, triethylamine and tert-butylamine.

Preferably, the invention relates to a compound of formula (I): 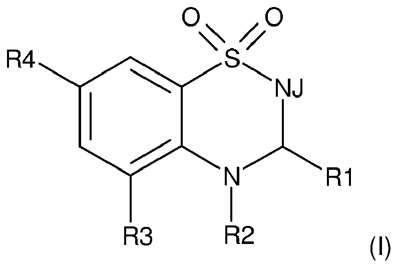 an enantiomer, diastereoisomer, or salt thereof with a pharmaceutically acceptable acid or base, wherein:
J, if present, is H,
R1 and R2 are, independently of each other, H or C₁-C₃ alkyl,
R3 is a heterocyclic or aryl group, where said heterocyclic group is monocyclic, aromatic or aliphatic, comprising at least one heteroatom selected from nitrogen, oxygen or sulphur, and said aryl group is monocyclic aromatic, or is a heterocyclic or aryl group substituted with at least one substituent group selected from C₁-C₃ alkyl, halogen, -OH, -CN, -COOH, - NO₂, -OY1, -COOY1, -NY1Y2, -(SO₂)NY1Y2, where Y1 and Y2 are, independently of each other, H or C₁-C₃, and
R4 is H, C₁-C₆ alkyl linear or branched, halogen, -CN, -NO₂, -OY1, -COOY1, -NY1 Y2, -(SO₂)NY1Y2, where Y1 and Y2 are, independently of each other, H or C₁-C₆ alkyl linear or branched.

More preferably, the invention relates to a compound of formula I: 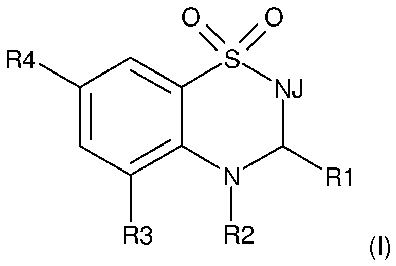 an enantiomer, diastereoisomer, or salt thereof with a pharmaceutically acceptable acid or base, wherein:
J, if present, is H,
R1 is H or C₁-C₃ alkyl,
R2 is H,
R3 is a 5- or 6-membered heterocyclic or aryl group or a 5- or 6-membered heterocyclic or aryl group substituted with at least one substituent group selected from C₁-C₃ alkyl, halogen, -OY1, -COOY1, -NY1Y2, where Y1 and Y2 are, independently of each other, H or C₁-C₃, and
R4 is halogen, C₁-C₃ alkyl, -NO₂ -OY1, -COOY1 or -NY1Y2, where Y1 and Y2 are, independently of each other, H or C₁-C₃.

According to a preferred embodiment, the compound has the formula (I) wherein R3 is an aromatic ring with 5 or 6 members comprising at least one heteroatom. Within this preferred embodiment, the following compounds are even more preferred:
1) 7-chloro-5-(3-furyl)-3-methyl-3,4-dihydro-2*H*-1,2,4-benzothiadiazine 1,1-dioxide 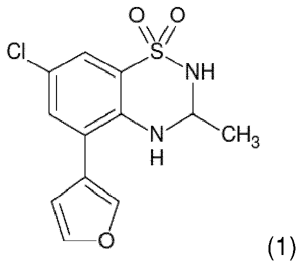
2) 7-chloro-3-methyl-5-thien-3-yl-3,4-dihydro-2*H*-1,2,4-benzothiadiazine 1,1-dioxide 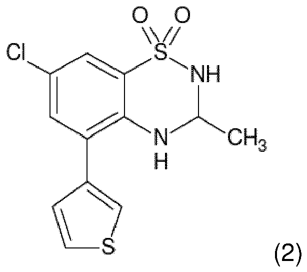
3) 7-chloro-5-(2-furyl)-3-methyl-3,4-dihydro-2*H*-1,2,4-benzothiadiazine 1,1-dioxide 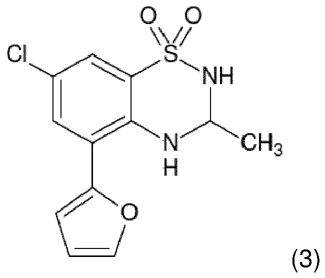
4) 7-chloro-5-(2-furyl)-3-ethyl-3,4-dihydro-2*H*-1,2,4-benzothiadiazine 1,1-dioxide 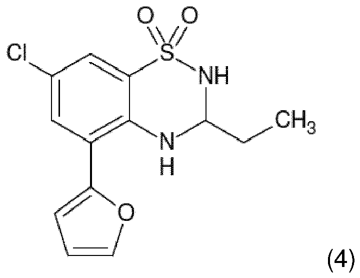
5) 7-chloro-5-(2-furyl)-3,4-dihydro-2*H*-1,2,4-benzothiadiazine 1,1-dioxide 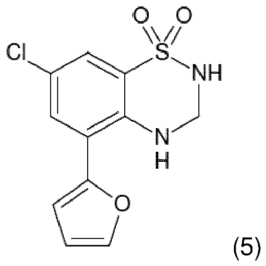
6) 7-chloro-5-(2-furyl)-3-methyl-1,2,4-benzothiadiazine 1,1-dioxide 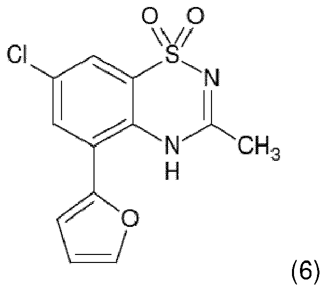
7) 7-chloro-3-methyl-5-thien-2-yl-3,4-dihydro-2*H*-1,2,4-benzothiadiazine 1,1-dioxide

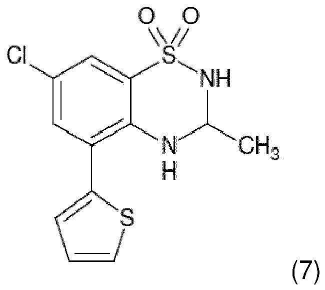

According to another preferred embodiment, the compound has the formula (I), wherein R3 is a 5- or 6-membered aromatic ring substituted with at least one substituent group selected from -OY1, -COOY1 and -NY1Y2, where Y1 and Y2 are, independently of each other, H or C₁-C₃.

Within this preferred embodiment, the following compounds are even more preferred:
8) 3-(7-chloro-3-methyl-1,1-dioxide-3,4-dihydro-2*H*-1,2,4-benzothiadiazine-5-yl) benzoic acid 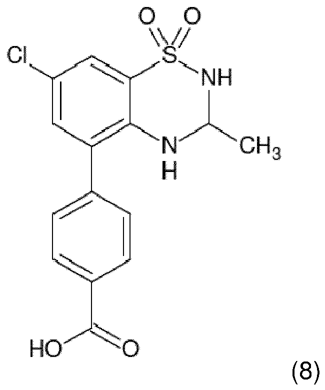
9) 3-(7-chloro-3-methyl-1,1-dioxide-3,4-dihydro-2*H*-1,2,4-benzothiadiazine-5-yl)aniline

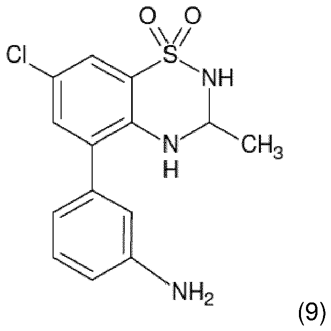

In another aspect, the present invention relates to a process for the preparation of the compound of formula (I), as described above, comprising the steps of:
a) halogenation at position 3 of the compound of formula (i) 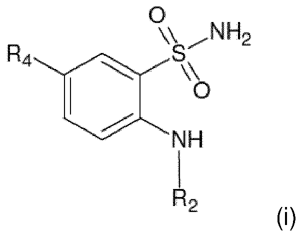
b) condensation by reaction with B(OH)₂R3, and
c) cyclisation by reaction with R1COH.

According to one embodiment, in the process of the invention, the step c) is performed before the step b).

In particular, the compound of formula (i) 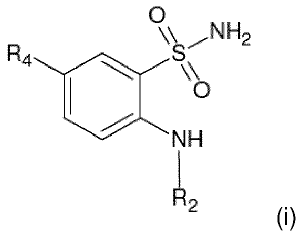 is halogenated in position 3 to form the compound (ii) 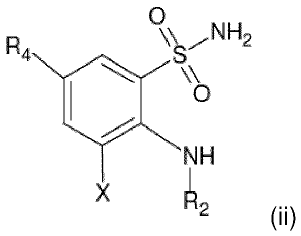 where X is a halogen selected from chlorine, iodine and bromine.

Subsequently, the compound (ii) is subjected to a Suzuki-coupling reaction with a boronic acid of with the formula B(OH)₂R3 to give the product (iii):

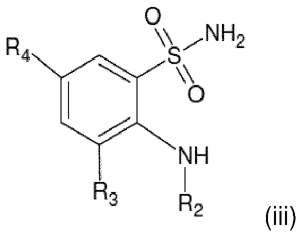

Finally, the compound (iii) is cyclised by reaction with R1COH to form the compound of formula (I).

Alternatively, the compound (ii) 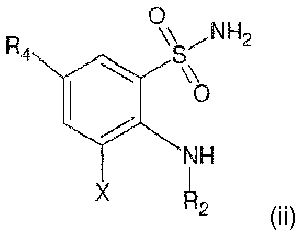 is first cyclised by reaction with R1 COH to form the compound of formula (iv): 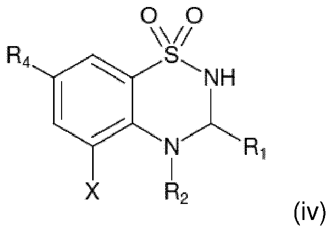 and subsequently subjected to a Suzuki-coupling reaction with a boronic acid of with the formula B(OH)₂R3 to give the compound of formula (I).

In a further aspect, the present invention relates to the use of the compound of formula (I) as an allosteric modulator of the AMPA receptor in the treatment of mnemocognitive disorders ascribable to an alteration of glutamatergic neurotransmission, such as age-related disorders, anxiety, progressive neurodegenerative disorders, depressive disorders, schizophrenia, obsessive-compulsive disorders and bipolar disorder.

As will be better illustrated by the following examples, the compound of formula (I) has in fact surprisingly proven to be capable of intervening on the AMPA receptor, in some cases by potentiating the synaptic currents induced by glutamate and minimising desensitisation, thus finding particularly advantageous application in the treatment of diseases such as age-related disorders, anxiety, progressive neurodegenerative disorders, depressive disorders and schizophrenia, and in other cases by reducing the synaptic currents induced by glutamate and increasing desensitisation, thus finding particularly advantageous application in the treatment of diseases such as obsessive-compulsive disorders and bipolar disorder.

In yet another aspect, the present invention relates to a pharmaceutical composition comprising at least one compound of formula (I) and suitable pharmaceutically acceptable excipients.

Working examples of the preparation of compounds according to the present invention are herein below provided for illustrative and non-limiting purposes, as well as examples of evaluation of their efficacy.

### EXAMPLES

### Example 1.

Preparation of 7-chloro-5-(3-furyl)-3-methyl-3,4-dihydro-2*H*-1,2,4-benzothiadiazine 1,1-dioxide

### Step a) Preparation of 5-chloro-3-iodo-2-aminobenzensulfonamide:

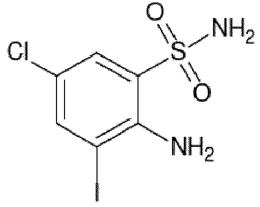

To a solution of 5-chloro-2-aminobenzensulfonamide (1.0 mmol) in 3 ml of DMF with magnetic stirring, a solution of CI-I (1.5 mmol) in 3 ml of glacial acetic acid was added dropwise. The mixture was left being stirred for 48h at room temperature. The mixture was then neutralised with a solution of NaOH 5M and extracted 3 times with ethyl acetate. The organic phase obtained was dehydrated with anhydrous Na₂SO₄ and the solvent was evaporated. Via column chromatography (ethyl acetate/petroleum ether), the pure compound was obtained (molar yield 72%).

mp 184-186 °C from petroleum ether

IR(nujol) 3401, 3363, 3267, 2725, 1598, 1612, 1300, 1147 cm⁻¹

¹H NMR (400MHz, DMSO) δ= 5.89 (s, broad, 2H), 7.60 (d, J=2.4Hz, 1H), 2.95 (m, 2H), 7.65 (s, broad, 2H), 7.90 (d, J=2.4H, 1H)

GC-MS (70eV) *m*/*z*= 332 (100)[M+], 315 (70), 251 (75), 124 (35).

HRMS-ESI: calc. for C₆H₇CIIN₂O₂S [M+H]⁺ 333.5539; found: 333.5541

### Step b) Preparation of 5-chloro-3-(3-furyl)-2-aminobenzensulfonamide

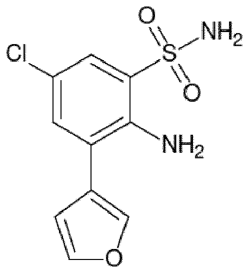

To a solution of 5-chloro-3-iodo-2-aminobenzensulfonamide (1.0 mmol) and 3-furanylboronic acid (1.1 mmol) in water/dioxane, tetrakis (triphenylphosphine) palladium (5% mol.) and Na₂CO₃ (10 mmol) were added. The reaction mixture was placed under reflux for 3h. The mixture was then neutralised with a solution of dil. HCl and extracted with ethyl acetate. The combined organic phases were dehydrated with anhydrous Na₂SO₄ and evaporated. The resulting solid was purified by chromatography to obtain the pure compound (molar yield 98%).

mp 81-83°C from petroleum ether

IR(nujol) 3393, 3283, 2348, 1633, 1303, 1322, 1113, 1073 cm⁻¹

¹H NMR (400MHz, CDCl₃) δ= 5.10 (s, broad, 4H), 6.56 (s, 1H),7.32 (d, J=2.5Hz, 1H); 7.57 (app. t, J=1.5Hz, 1H), 7.63 (s, 1H), 7.75 (d, J=2.5Hz, 1H)

¹³C NMR (400MHz, CDCl₃) δ=110.6, 120.5, 122.5, 123.0, 126.0, 134.4, 140.6, 141.5, 144.3

GC-MS (70eV) *m*/*z* = 272 (84)[M+], 191 (67), 163 (60), 128 (100), 101 (30) HRMS-ESI: calc. for C₁₀H₁₀ClN₂O₃S [M+H]⁺ 273.7154; found: 273.7156 Step c) Preparation of 7-chloro-5-(3-furyl)-3-methyl-3,4-dihydro-2*H*-1,2,4-benzothiadiazine 1,1-dioxide (1)

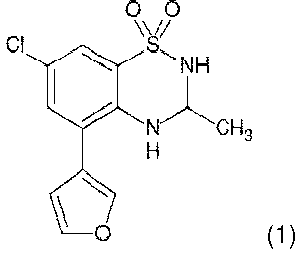

To a solution of 5-chloro-3-(3-furyl)-2-aminobenzensulfonamide (1.0 mmol) in isopropanol with magnetic stirring, acetaldehyde (4.0 mmol) and ethyl acetate saturated with HCl (3-4 drops) were added. The reaction mixture was maintained at 65°C for around two hours. The mixture was then concentrated in the rotavapor and, after adding water, was extracted with ethyl acetate. The organic phases were then dehydrated with Na₂SO₄ and evaporated in the rotavapor, obtaining the pure compound (1) (99% molar yield).

mp 183-185°C from petroleum ether

IR(nujol) 3337, 3283, 2730, 2358, 1571, 1328, 1296, 1167, 1085 cm⁻¹

¹H NMR (400MHz, CDCl₃) δ= 1.54 (d, J=6.1, 3H), 4.65 (s, broad, 1H), 4.69 (s, 1H) 5.03 (s, broad, 1H), 6.55 (s, 1H), 7.25 (d, J=2.5Hz, 1H), 7.53 (d, J=2.5Hz, 1H), 7.58 (app.t, 1.35, 1H), 7.63 (s, 1H)

¹³C NMR (400MHz, CDCl₃) δ=20.9, 62.5, 110.3, 120.6, 121.7, 123.3, 133.5, 136.5, 137.8, 138.9, 140.6, 144.4

GC-MS (70eV) *m*/*z* = 298 (100) [M+], 283 (57), 192 (98), 163 (55), 128 (77), 101 (35)

HRMS-ESI: calc. for C₁₂H₁₂ClN₂O₃S [M+H]⁺ 299.7527; found: 299.7527

### Example 2.

Preparation of 7-chloro-5-thien-3-yl-3-methyl-3,4-dihydro-2*H*-1,2,4-benzothiadiazine 1,1-dioxide

### Step b) Preparation of 5-chloro-3-thien-3-yl-2-aminobenzensulfonamide

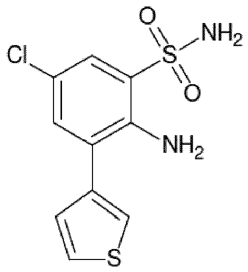

Starting from the compound of step a) of Example 1, namely 5-chloro-3-iodo-2-aminobenzensulfonamide, it was proceeded as described in step b) of Example 1, using in this case 3-thiophene boronic acid instead of 3-furanylboronic acid (molar yield = 89%).

mp 174-176°C from petroleum ether

IR(nujol) 3365, 3288, 2723, 2359, 1619, 1559, 1300, 1143 cm⁻¹

¹H NMR (400MHz, CDCl₃) δ= 4.97 (s, broad, 2H), 5.03 (s, broad, 2H), 7.17 (d, J=4.8Hz, 1H), 7.34 (d, J=2.3Hz, 1H); 7.38 (m, 1H), 7.51 (m, 1H), 7.78 (d, J= 2.3Hz, 1H)

¹³C NMR (400MHz, CDCl₃) δ= 122.6, 124.3, 126.3, 127.0, 127.5, 127.8, 134.6, 136.5, 136.9, 141.0

GC-MS (70eV) *m*/*z* = 288 (62)[M+], 283 (57), 207 (70), 172 (100), 145 (15) HRMS-ESI: calc. for C₁₀H₁₀CIN₂O₂S₂ [M+H]⁺ 289.7810; found: 289.7808.

### Step c) Preparation of 7-chloro-5-thien-3-yl-3-methyl-3,4-dihydro-2H-1,2,4-benzothiadiazine 1,1-dioxide

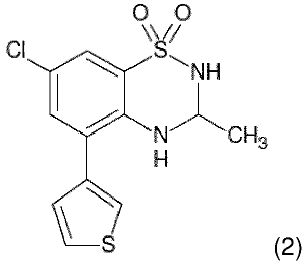

Using the compound obtained in the previous step b), it was proceeded as in step c) of Example 1, thus obtaining the pure compound (2) (molar yield = 98%).

mp 140-142°C from hexane

IR(nujol) 3391, 3196, 2359,1593, 1318, 1305, 1161, 1134 cm^{-1 1}H NMR (200MHz, CDCl₃) δ= 1.52 (d, J= 6.1 Hz, 3H), 4.55 (d, J= 12.9Hz, 1H), 4.71 (s, broad, 1H), 5.04 (m, 1H), 7.17 (dd, J=4.9Hz,1.3Hz, 1H), 7.27 (d, J=2.4Hz, 1H); 7.39 (dd, J=1.3Hz, 2.9Hz, 1H), 7.52(m, 1H), 7.59 (d, J= 2.4Hz, 1H)

¹³C NMR (400MHz, CDCl₃) δ= 122.6, 124.3, 126.3, 127.0, 127.5, 127.8, 134.6, 136.5, 136.9, 141.0

GC-MS (70eV) *m*/*z*=314 (76)[M+], 299 (45), 207 (57), 173 (100), 145 (25) HRMS-ESI: calc. for C₁₂H₁₂ClN₂O₂S₂ [M+H]⁺ 315.8183; found: 315.8186. Example 3.

Preparation of 1) 7-chloro-5-(2-furyl)-3-methyl-3,4-dihydro-2*H*-1,2,4-benzothiadiazine 1,1-dioxide

### Step b) Preparation of 5-chloro-3-(2-furyl)-2-aminobenzensulfonamide

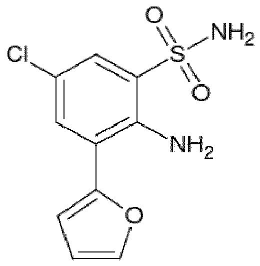

Starting from the compound of step a) of Example 1, namely 5-chloro-3-iodo-2-aminobenzensulfonamide, it was proceeded as described in step b) of Example 1, using in this case 2-furanylboronic acid instead of 3-furanylboronic acid (molar yield = 90%).

mp 146-148°C from hexane

IR(nujol) 3393, 3353, 3253, 2723, 2359, 1626, 1330, 1157, 1136 cm⁻¹

¹H NMR (400MHz, CDCl₃) δ= 5.04 (s, broad, 2H), 5.62 (s, broad, 2H), 6.57 (s, 1H), 6.66 (d, J=3.3Hz, 1H), 7.56 (s, 1H), 7.58 (d, J=2.1 Hz, 1H), 7.75 (d, J=2.1 Hz, 1H) ¹³C NMR (400MHz, CDCl₃) δ=108.9, 111.8, 120.3, 122.6, 127.3, 132.2, 140.2, 142.7, 150.4

GC-MS (70eV) *m*/*z*= 272 (84) [M+], 191 (30), 163 (32), 128 (100), 101 (23) HRMS-ESI: calc. for C₁₀H₁₀ClN₂O₃S [M+H]⁺ 273.7154; found: 273.7158

### Step c) Preparation of 7-chloro-5-(2-furyl)-3-methyl-3,4-dihydro-2H-1,2,4-benzothiadiazine 1,1-dioxide

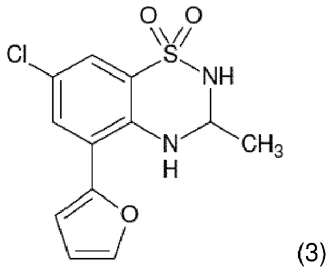

Using the compound obtained in the previous step b), it was proceeded as in step c) of Example 1, thus obtaining the pure compound (3) (molar yield = 98%).

mp 195-197°C from petroleum ether

IR(nujol) 3385, 3262, 2359, 1619, 1585, 1559, 1330, 1300, 1143 cm⁻¹

¹H NMR (400MHz, CDCl₃) δ= 1.62 (d, J= 6.1 Hz, 3H), 4.51 (d, J= 13Hz, 1H), 5.11 (m, 1H), 5.87 (s, 1H, broad), 6.56 (dd, J= 1.8Hz, 3.3Hz, 1H), 6.65 (d, J= 3.4Hz, 1H), 7.50 (d, J= 3.4Hz, 1H), 7.55 (d, J= 1.3Hz, 1H), 7.59 (d, J= 2.3Hz, 1H)

¹³C NMR (400MHz, CDCl₃) δ=21.1, 62.4, 108.9, 111.9, 118.9, 123.2, 123.9, 124.0, 131.1, 137.6, 142.7, 150.5

GC-MS (70eV) *m*/*z* = 298 (100) [M+], 283 (67), 255 (17), 192 (52), 164 (35), 128 (68)

HRMS-ESI: calc. for C₁₂H₁₂ClN₂O₃S [M+H]⁺ 299.7527; found: 299.7529

### Example 4.

Preparation of 7-chloro-5-(2-furyl)-3-ethyl-3,4-dihydro-2*H*-1,2,4-benzothiadiazine 1,1-dioxide

### Step c) Preparation of 7-chloro-5-(2-furyl)-3-methyl-3,4-dihydro-2H-1,2,4-benzothiadiazine 1,1-dioxide

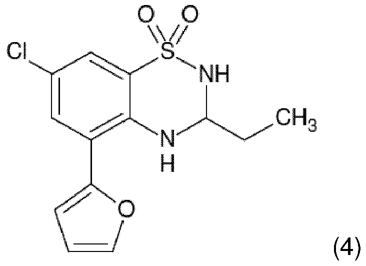

To a solution of 5-chloro-3-(2-furyl)-2-aminobenzensulfonamide (1.00 mmol), obtained as in step b) of Example 3, in isopropanol (10 ml) with magnetic stirring, propionaldehyde (5 mmol) and ethyl acetate saturated with HCl (3-4 drops) were added. The reaction mixture was maintained at 65°C for around two hours, then concentrated in the rotavapor and water was added. After extraction with ethyl acetate, the organic phases were dehydrated with anhydrous Na₂SO₄ and evaporated, obtaining the pure compound (4) (molar yield 97%).

mp 148-150°C from petroleum ether

IR(KBr) 3404, 3300, 2924, 2853, 1735, 1588, 1489, 1333, 1168 cm⁻¹

¹H NMR (400MHz, CDCl₃) δ= 1.14 (t, J=7.5, 3H), 1.90 (m, 2H), 4.51 (s, broad, 1H), 4.91 (m, 1H) 5.93 (s, broad, 1H), 6.56 (dd, J= 1.8Hz, 3.3Hz, 1H), 6.65 (d, J=3.4Hz, 1H), 7.48 (d, J=3.4Hz, 1H), 7.55 (d, J=1.4Hz, 1H) 7.56 (d, J=2.4Hz, 1H)

¹³C NMR (400MHz, CDCl₃) δ=8.8, 28.1, 67.1, 108.9, 111.9, 118.9, 123.0, 123.9, 124.1, 131.1, 137.7, 142.6, 150.6

GC-MS (70eV) *m*/*z*= 312 (30) [M+], 283 (100), 192 (20), 156 (15), 128 (25) HRMS-ESI: calc. for C₁₃H₁₄CIN₂O₃S [M+H]⁺ 313.7793; found 313.7794

### Example 5.

Preparation of 7-chloro-5-(2-furyl)-3,4-dihydro-2*H*-1,2,4-benzothiadiazine 1,1-dioxide

### Step c) Preparation of 7-chloro-5-(2-furyl)-3-methyl-3,4-dihydro-2H-1,2,4-benzothiadiazine 1,1-dioxide

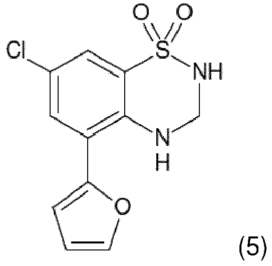

To a solution of 5-chloro-3-(2-furyl)-2-aminobenzensulfonamide (1.00 mmol), obtained as in step b) of Example 3, in isopropanol (10 ml) with magnetic stirring, formaldehyde (3 mmol) and ethyl acetate saturated with HCl (3-4 drops) were added. The reaction mixture was maintained at 65°C for around two hours, then concentrated in the rotavapor and water was added. After extraction with ethyl acetate, the organic phases were dehydrated with anhydrous Na₂SO₄ and evaporated in the rotavapor. The product was then purified by means of column chromatography (ethyl acetate/petroleum ether), thus obtaining the pure compound (molar yield 53%).

mp 204-206 °C from petroleum ether

IR(KBr) 3439, 3325, 2926, 2853, 2354, 1724, 1496, 1462, 1344, 1165 cm⁻¹

¹H NMR (400MHz, CDCl₃) δ= 4.71 (s, broad, 1H), 4.90 (s, 2H), 6.06 (s, broad, 1H), 6.57 (dd, J= 1.8Hz, 3.3Hz, 1H), 6.66 (d, J=3.4Hz, 1H), 7.47 (d, J=3.4Hz, 1H), 7.55 (d, J=1.4Hz, 1H) 7.58 (d, J=2.4Hz, 1H)

¹³C NMR (400MHz, CDCl₃) δ= 55.4, 109.0, 111.9, 119.1, 123.2, 124.2, 124.9, 131.2, 137.6, 142.7, 150.5

GC-MS (70eV) *m*/*z* = 284 (100) [M+], 255 (30), 207 (76), 192 (74), 156 (34), 128 (64)

HRMS-ESI: calc. for C₁₁H₁₀ClN₂O₃S [M+H]⁺ 285.7261; found 285.7264

### Example 6.

Preparation of 7-chloro-5-(2-furyl)-3-methyl-1,2,4-benzothiadiazine 1,1-dioxide Step c) Preparation of 7-chloro-5-iodo-3-yl-3-methyl-3,4-dihydro-2*H*-1,2,4-benzothiadiazine 1,1-dioxide

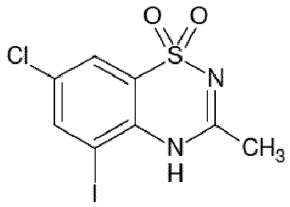

A solution of 5-chloro-3-iodo-2-aminobenzensulfonamide (1.00 mmol), obtained as in step a) of Example 1, in acetic anhydride (10 ml), was maintained at a temperature of 100°C for 2 hours. Subsequently, water was added (10ml) and the reaction mixture was neutralised. After extraction with ethyl acetate, the organic phase was dehydrated with anhydrous Na₂SO₄ and evaporated in the rotavapor. The product was then purified by means of column chromatography (ethyl acetate/petroleum ether), thus obtaining the pure compound (molar yield 93%).

mp 234-236°C from petroleum ether

IR(nujol) 3376, 3266, 2379, 1629, 1585, 1559, 1330 cm⁻¹

¹H NMR (400MHz, CDCl₃) δ= 1.90 (s, 1H), 6.01 (s, broad, 1H) 8.05 (d, J=2.3Hz, 1H) 8.13 (d, J=2.3Hz, 1H)

¹³C NMR (400MHz, CDCl₃) δ= 29.7, 104.1, 130.7, 136.0, 137.8 140.9, 142.7, 176.8

GC-MS (70eV) *m*/*z*= 356 (86) [M+], 315 (100), 251 (96), 124 (65), 88 (54)

HRMS-ESI: calc. for C₈H₇CllN₂O₂S [M+H]⁺ 357.5753; found 357.5756

### Step b) Preparation of 7-chloro-5-(2-furyl)-3-methyl-1,2,4-benzothiadiazine 1,1-dioxide

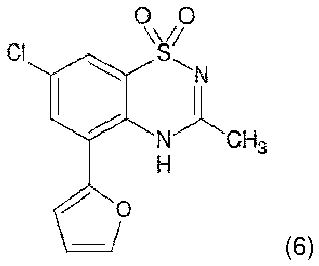

Using the compound obtained in the previous step c), it was proceeded as in step b) of Example 1, in this case using 2-furanylboronic acid instead of 3-furanylboronic acid. The pure compound (6) was obtained (molar yield = 83%).

mp 120-123°C from hexane

IR 3382, 3261, 2921, 1580, 1170, 1137

NMR ¹H NMR (400MHz, Acetone_{d6}) δ= 1.92 (s, 3H), 6.54 (dd, J=1.9 Hz, 3.3Hz, 1H), 6.95 (d, J= 3.4Hz, 1H), 7.65 (d, J= 1.4Hz, 1H), 7.93 (d, J= 2.5Hz, 1H), 7.96 (d, J= 2.5Hz, 1H)

¹³C NMR (400MHz, Acetone_{d6}) δ= 29.4, 110.6, 112.0, 129.0, 130.6, 131.2, 132.1, 133.4, 140.2, 143.3, 149.0, 169.7

GC-MS (70eV) *m*/*z*= 296 (100) [M+], 255 (40), 191 (92), 198 (98)

HRMS-ESI: calc. for C₁₂H₁₀ClN₂O₃S [M+H]⁺ 297.7368; found 297.7370

### Example 7.

Preparation of 7-chloro-5-thien-2-yl-3-methyl-3,4-dihydro-2*H*-1,2,4-benzothiadiazine 1,1-dioxide

### Step b) Preparation of 5-chloro-3-thien-2-yl-2-aminobenzensulfonamide

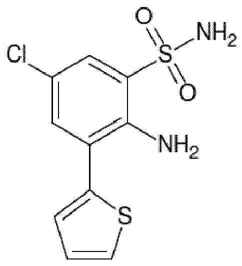

Starting from the compound of step a) of Example 1, namely 5-chloro-3-iodo-2-aminobenzensulfonamide, it was proceeded as described in step b) of Example 1, using in this case 2-thiopheneboronic acid instead of 3-furanylboronic acid (molar yield = 91%).

mp 80-82 °C from hexane

IR(KBr) 3368, 3268, 2920, 2851, 2371, 1720, 1615, 1458, 1319, 1144 cm⁻¹

¹H NMR (400MHz, CDCl₃) δ= 5.16 (s, broad, 2H), 5.2 (s, broad, 2H), 7.22 (m, 2H),7.39 (d, J=2.4Hz, 1H), 7.45 (dd, J= 4.9Hz, 4.8Hz, 1H), 7.77 (d, J= 2.4Hz, 1H) ¹³C NMR (400MHz, CDCl₃) δ= 122.3, 124.3, 126.2, 127.1, 127.5, 127.6, 127.9, 135.3, 136.9, 141.3

GC-MS (70eV) m/z= 288 (67) [M+], 207 (100), 172 (95), 145(17)

HRMS-ESI: calc. for C₁₀H₁₀ClN₂O₂S₂ [M+H]⁺ 289.7810; found: 289.7812

### Step c) Preparation of 7-chloro-5-thien-2-yl-3-methyl-3,4-dihydro-2H-1,2,4-benzothiadiazine 1,1-dioxide

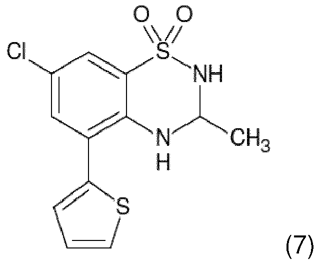

Using the compound obtained in the previous step b), it was proceeded as in step c) of Example 1, thus obtaining the pure compound (7) (molar yield = 99%).

mp 171-173°C from hexane

IR(KBr) 3405, 3314, 3031, 2927, 2359, 1580, 1485, 1340, 1171, 1142 cm⁻¹

¹H NMR (400MHz, CDCl₃) δ= 1.51 (d, J= 6.1 Hz, 3H), 4.69 (d, J= 12.8Hz, 1H), 4.97 (s, broad, 1H), 5.01 (m, 1H), 7.17 (m, 2H), 7.32 (d, J=2.3Hz, 1H), 7.45 (dd, J=4.9Hz, 4.8Hz 1H), 7.58 (d, J= 2.3Hz, 1H)

¹³C NMR (400MHz, CDCl₃) δ= 29.6, 62.4, 123.0, 123.2, 124.0, 127.3, 127.4, 127.5, 128.0, 134.5, 136.6, 138.9

GC-MS (70eV) *m*/*z* = 314 (100) [M+], 299 (65), 207 (67), 172 (90), 145 (21), 128(16)

HRMS-ESI: calc. for C₁₂H₁₂ClN₂O₂S₂ [M+H]⁺ 315.8183; found: 315.8188

### Example 8.

Preparation of 3-(7-chloro-3-methyl-1,1-dioxide-3,4-dihydro-2*H*-1,2,4-benzothiadiazine-5-yl) benzoic acid

### Step b) Preparation of 5'-chloro-3'-(-aminosulfonyl)-2'-amino-1,1'-biphenyl-4-carboxylic acid

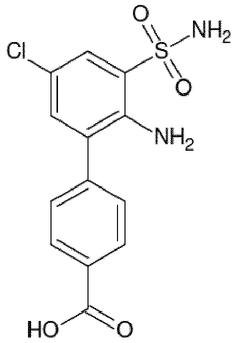

Starting from the compound of step a) of Example 1, namely 5-chloro-3-iodo-2-aminobenzensulfonamide, it was proceeded as described in step b) of Example 1, using in this case 4-carboxyphenylboronic acid instead of 3-furanylboronic acid (molar yield = 95%).

mp 216-218°C from petroleum ether

IR(KBr) 3485, 3373, 3275, 2359, 1691, 1609, 1307, 1148 cm⁻¹

¹H NMR (400MHz, CD₃OD) δ= 4.97 (s, broad, 4H), 7.22 (d, J=2.4Hz, 1H),7.52 (d, J=8.1 Hz, 2H), 7.73 (d, J=2.4Hz , 1H), 8.01 (d, J=8.1 Hz 2H)

¹³C NMR (400MHz, CD₃OD) δ= 122.1, 128.1, 128.7, 130.1, 130.2, 130.5, 131.6, 131.8, 134.2, 134.8, 142.5, 143.3, 169.4

HRMS-ESI: calc. for C₁₃H₁₂ClN₂O₄S [M+H]⁺ 327.7628; found: 327.7629

### Step c) Preparation of 3-(7-chloro-3-methyl-1,1-dioxide-3,4-dihydro-2H-1,2,4-benzothiadiazine-5-yl) benzoic acid

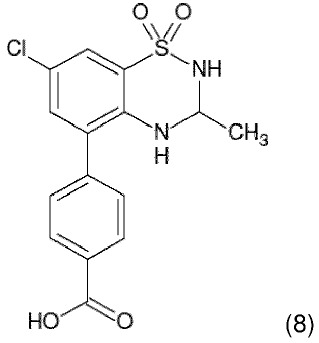

Using the compound obtained in the previous step b), it was proceeded as in step c) of Example 1, thus obtaining the pure compound (8) (molar yield = 97%).

mp 264-266°C from hexane

IR(KBr) 3456, 3420, 2960, 2924, 1702, 1595, 1488, 1261, 1097, 1018 cm⁻¹

¹H NMR (400MHz, CD₃OD) δ= 1.30 (d, J=6.0Hz, 3H), 4.82 (s, broad, 1H), 4.91 (m, 1H), 5.33 (s, broad, 1H), 6.9 (d, J=2.3 Hz, 1H), 7.17 (d, J=7.8Hz, 2H), 7.37 (d, J=2.3Hz, 1H), 8.00 (d, J=7.8Hz 2H)

¹³C NMR (400MHz, CD₃OD) δ= 32.7, 63.7, 122.4, 124.0, 124.9, 129.7, 131.5, 133.9, 134.1, 140.1, 140.4, 170.4

HRMS-ESI: calc. for C₁₅H₁₄ClN₂O₄S [M+H]⁺ 353.8001; found: 353.8004

### Example 9.

Preparation of 3-(7-chloro-3-methyl-1,1-dioxide-3,4-dihydro-2*H*-1,2,4-benzothiadiazine-5-yl)aniline

### Step c) Preparation of 7-chloro-5-iodo-3-methyl-3,4-dihydro-2H-1,2,4-benzothiadiazine 1,1-dioxide

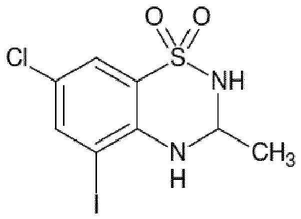

To a solution of 5-chloro-3-iodo-2-aminobenzensulfonamide (1.0 mmol), obtained as in step a) of Example 1, in isopropanol with magnetic stirring, acetaldehyde (4.0 mmol) and ethyl acetate saturated with HCl (3-4 drops) were added. The reaction mixture was maintained at 65°C for around 2h and then extracted with ethyl acetate. The organic phase was then dehydrated with Na₂SO₄ and the pure compound (98% molar yield) was obtained.

mp 230-232°C from hexane

IR 3378, 3266, 2924, 1580, 1170, 1137

¹H NMR (400MHz, CDCl₃) δ= 1.58 (d, J= 6.1 Hz, 3H), 4.32 (s, broad, 1H), 4.84 (s, broad, 1H), 5.14 (q, J= 6.1 Hz, 1H), 7.65 (d, J= 2.2Hz, 1 H), 7.77 (d, J= 2.2Hz, 1H) GC-MS (70eV) *m*/*z*=358 (77) [M+], 343 (100), 252 (80), 125 (35)

HRMS-ESI: calc. for C₈H₉CllN₂O₂S [M+H]⁺ 359.5912; found: 359.5914

### Step b) Preparation of 3-(7-chloro-3-methyl-1,1-dioxide-3,4-dihydro-2H-1,2,4-benzothiadiazine-5-yl)aniline

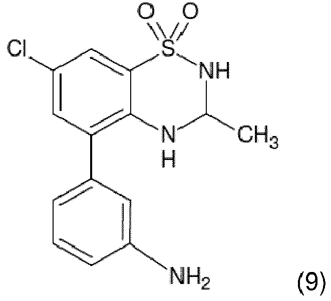

Using the compound obtained in the previous step c), it was proceeded as in step b) of Example 1, in this case using 3-aminophenylboronic acid instead of 3-furanylboronic acid. The pure compound (9) was obtained (molar yield = 50%).

mp 81-88°C from petroleum ether

IR(KBr): 3400, 3312, 3021, 2920, 2379, 1590, 1485, 1340cm⁻¹

¹H NMR (400MHz, CDCl₃) δ= 1.46 (d, J=6.1Hz, 3H), 3.80 (s, broad, 2H), 4.49 (s, broad, 1H), 4,67 (s, broad, 2H), 5.01 (m, 1H), 6.60 (s, 1H); 6.68 (d, J= 8.0Hz, 1H), 6.72 (dd, J= 8.0Hz, 2.1 Hz, 1H), 7.18 (d, J=2.4Hz, 1H), 7.24 (t, J=8.0Hz, 1H) 7.69 (d, J=2.4Hz 1H)

¹³C NMR (400MHz, CDCl₃) δ=20.9, 62.5, 115.1, 115.3, 118.4, 122.7, 123.1, 123.3, 130.4, 130.8, 133.7, 136.8, 138.5, 147.4 GC-MS (70eV) *m*/*z*=323 (95)[M+], 308[75], 280(17) 216 (51), 181 (100), 154 (34) HRMS-ESI: calc. for C₁₄H₁₅ClN₃O₂S [M+H]⁺ 324.8052; found: 324.8053

### Example 10.

Evaluation of the pharmacological activity of the compounds according to the invention

The pharmacological studies were conducted using the patch-clamp technique on primary cultures of cortical neurons.

The primary neuronal cultures were obtained from 1-day-old Sprague-Dawley rats. The cells were dispersed with trypsin (0.24 mg/ml) and plated out at a density of 10⁶ cells/ml on 35mm Petri dishes pretreated with poly-L-lysine (10 µg/ml). The cells were cultivated in Eagle's Basal Medium with the addition of 10% foetal calf serum, 2 mM glutamine and 100 µg/ml gentamicin, and stored in an incubator at 37°C in 5% carbon dioxide.

To prevent astroglial proliferation, cytosine arabinofuranoside was added (1-10 υM) to the cultures 24h after plating out.

The patch-clamp recordings were performed on a single cell after 7 days in culture using the voltage-clamp technique in whole-cell configuration (Hamill OP, Marty A, Neher E, Sakmann B, Sigworth FJ (1981): Improved patchclamp techniques for high-resolution current recording from cell and cell-free membrane patches. Pflugers Arch, 85-100). The borosilicate glass electrodes were obtained with a vertical puller (PB-7, Narishige, Japan) and had a resistance of 5-7 MOhm when filled with intracellular solution. The currents were amplified with an Axopatch 1 D amplifier (Axon Instruments, Foster City, CA), filtered at 5 kHz and digitised at 10 kHz using the pClamp software (Axon Instruments, Foster City, CA).

The intracellular solutions contained: KCI 140mM, MgCl₂ 3mM, EGTA 5mM, HEPES 5mM, ATP-Na 2mM, and were brought to pH 7.3 with KOH. The cells were continuously perfused with an external solution containing: NaCl 145mM, KCI 5mM, CaCl2 1mM, HEPES 5mM, glucose 5mM, saccharose 20Mm, and brought to pH 7.4 with NaOH.

The compounds tested were dissolved in DMSO and diluted to the final concentration in the extracellular solution. (The DMSO in the final concentration is less than 0.1%.) The kainate (SIGMA) was dissolved in the extracellular solution. All of the substances were applied directly by gravity through a "Y-tube" perfusion system (Murase K, Ryu PD, Randic M (1989). Excitatory and inhibitory amino acids and peptide-induced responses in acutely isolated rat spinal dorsal horn neurons. Neurosci Lett 103, 56-63).

The electrophysiological data was analysed using the software pCLAMP 6.3 (Axon Instruments).

The compounds according to the invention have proven to be potent allosteric modulators of the response mediated by AMPA-kainate receptors. The currents evoked by the application of kainic acid (KA) were significantly modified by the compounds according to the invention, as shown in Table 1 below, which shows the percentage changes in the KA currents in rat cortical neurons in the presence of certain compounds according to the invention at the concentration of 10 µM. The values are expressed as the mean ± SE (n = 4-6).

In particular, the data of Table 1 showed that compound (1) resulted to be one of the most active and it was therefore studied further. In fact, compound (1) increased the current induced by Kainate by around 360 times, thus acting as a positive allosteric modulator, in the same way as compounds 2, 3 and 5. Compounds 1, 2, 3 and 5 therefore intensified the activation of the channel. Compounds 1, 2, 3 and 5 therefore showed the capability, even at very low concentrations (10 µM), to potentiate the current induced by Kainate. Such compounds have thus been advantageously used in diseases such as age-related disorders, anxiety, progressive neurodegenerative disorders, depressive disorders and schizophrenia.

On the other hand, compounds 4 and 6 acted as negative allosteric modulators, which induced a decrease in the channel activity. Compounds 4 and 6 modulated the activity of the AMPA receptor, increasing desensitisation and therefore decreasing the current induced by Kainate. Such compounds were used in diseases in which there is an over-activation of the AMPA receptor, as in obsessive-compulsive disorders and bipolar disorders.

**Table 1**

| Compound | Variation in the current induced by Kainate, % |
|---|---|
| (1) | 358±80 |
| (2) | 214±41 |
| (3) | 80±16 |
| (4) | -32±12 |
| (5) | 20±5 |
| (6) | -21±9 |

As mentioned, the compound (1) showed to be particularly active and was therefore studied further. In particular, Figure 1 is a graph showing the dose/response data of compound (1) according to the present invention, from which it was possible to calculate an EC50 of 1.1 µM and an EC2X (i.e. the concentration at which the AMPA receptor currents are potentiated 2 times) of 500 nM. This means that compound (1) according to the present invention showed the ability to act advantageously as a positive allosteric modulator of the AMPA receptor already at extremely low concentrations, i.e. it already showed a high activity in potentiation of the currents induced by Kainate at concentrations of 1 µM.

From the detailed description and the examples above, the advantages obtained through the use of the compounds according to the present invention are evident. In fact, it is observed that the compound of formula (I) acts as an allosteric modulator of the AMPA receptor, in some cases by potentiating the synaptic currents induced by glutamate and minimising desensitisation, thus finding particularly advantageous application in the treatment of diseases such as age-related disorders, anxiety, progressive neurodegenerative disorders, depressive disorders and schizophrenia, and in other cases by reducing the synaptic currents induced by glutamate and increasing desensitisation, thus finding particularly advantageous application in the treatment of diseases such as obsessive-compulsive disorders and bipolar disorder.

## Claims

1. Compound of formula I: 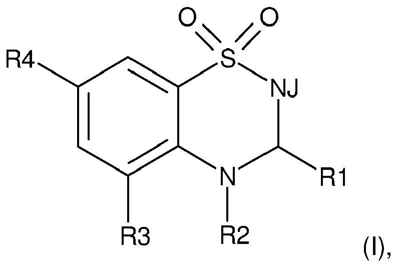 an enantiomer, diastereoisomer, or salt thereof with a pharmaceutically acceptable acid or base, wherein:
J, if present, is H,
R1 and R2 are, independently of each other, H, C₁-C₃ alkyl or C₁-C₃ alkyl substituted with at least one halogen,
R3 is a heterocyclic or aryl group, where said heterocyclic group is monocyclic or bicyclic, aromatic or aliphatic, comprising at least one heteroatom selected from nitrogen, oxygen or sulphur, and said aryl group is monocyclic or bicyclic, having at least one aromatic ring, or is a heterocyclic or aryl group substituted with at least one substituent group selected from C₁-C₃ alkyl, halogen, -OH, -CN, -COOH, -NO₂, -OY1, -COOY1, -NY1Y2, - (SO₂)NY1Y2, where Y1 and Y2 are, independently of each other, H or C₁-C₆ alkyl linear or branched, and
R4 is H, C₁-C₆ alkyl linear or branched, halogen, -OH, -CN, -COOH, -NO₂, - OY1, -COOY1, -NY1 Y2, -(SO₂)NY1Y2, where Y1 and Y2 are, independently of each other, H or C₁-C₆ alkyl linear or branched, or R4 is Z1, -OZ1, -OCOZ1 or -OCNZ1, where Z1 is a heterocyclic or aryl group, where said heterocyclic group is monocyclic or bicyclic, aromatic or aliphatic, comprising at least one heteroatom selected from nitrogen, oxygen or sulphur, and said aryl group is monocyclic or bicyclic, having at least one aromatic ring, or is a heterocyclic or aryl group substituted with at least one substituent group selected from C₁-C₃ alkyl, halogen, -OH, -CN, -COOH, -NO₂, -OY1, -COOY1, -NY1Y2, - (SO₂)NY1Y2, where Y1 and Y2 are, independently of each other, H or C₁-C₆ alkyl linear or branched.

2. The compound of claim 1, wherein:
J, if present, is H,
R1 and R2 are, independently of each other, H or C₁-C₃ alkyl,
R3 is a heterocyclic or aryl group, where said heterocyclic group is monocyclic, aromatic or aliphatic, comprising at least one heteroatom selected from nitrogen, oxygen or sulphur, and said aryl group is monocyclic aromatic, or is a heterocyclic or aryl group substituted with at least one substituent group selected from C₁-C₃ alkyl, halogen, -OH, -CN, -COOH, - NO₂, -OY1, -COOY1, -NY1Y2, -(SO₂)NY1Y2, where Y1 and Y2 are, independently of each other, H or C₁-C₃, and
R4 is H, C₁-C₆ alkyl linear or branched, halogen, -CN, -NO₂, -OY1, -COOY1, -NY1 Y2, -(SO₂)NY1Y2, where Y1 and Y2 are, independently of each other, H or C₁-C₆ alkyl linear or branched.

3. The compound of claim 2, wherein:
J, if present, is H,
R1 is H or C₁-C₃ alkyl,
R2 is H,
R3 is a 5- or 6-membered heterocyclic or aryl group or a 5- or 6-membered heterocyclic or aryl group substituted with at least one substituent group selected from C₁-C₃ alkyl, halogen, -CN, -NO₂, -OY1, -COOY1, -NY1Y2, - (SO₂)NY1Y2, where Y1 and Y2 are, independently of each other, H or C₁-C₃, and
R4 is halogen, C₁-C₃ alkyl, -OY1, -COOY1 or -NY1 Y2, where Y1 and Y2 are, independently of each other, H or C₁-C₃.

4. The compound of claim 3, selected from:
1) 7-chloro-5-(3-furyl)-3-methyl-3,4-dihydro-2*H*-1,2,4-benzothiadiazin 1,1-dioxide 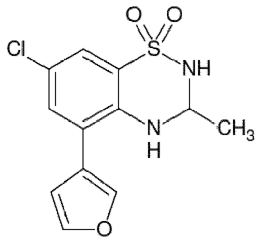
2) 7-chloro-3-methyl-5-thien-3-yl-3,4-dihydro-2*H*-1,2,4-benzothiadiazin 1,1-dioxide 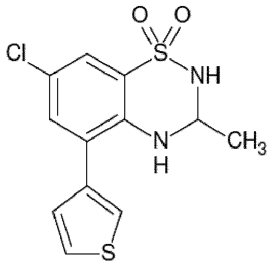
3) 7-chloro-5-(2-furyl)-3-methyl-3,4-dihydro-2*H*-1,2,4-benzothiadiazin 1,1-dioxide 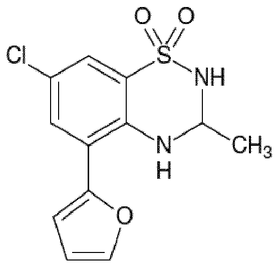
4) 7-chloro-5-(2-furyl)-3-ethyl-3,4-dihydro-2*H*-1,2,4-benzothiadiazin 1,1-dioxide 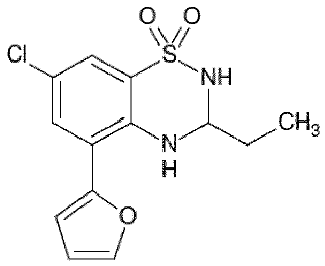
5) 7-chloro-5-(2-furyl)-3,4-dihydro-2*H*-1,2,4-benzothiadiazin 1,1-dioxide 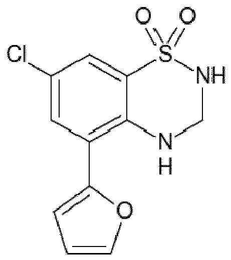
6) 7-chloro-5-(2-furyl)-3-methyl-1,2,4-benzothiadiazin 1,1-dioxide 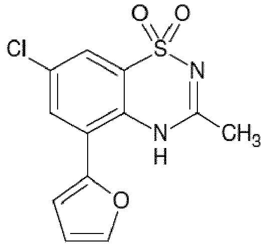
7) 7-chloro-3-methyl-5-thien-2-yl-3,4-dihydro-2*H*-1,2,4-benzothiadiazin 1,1-dioxide

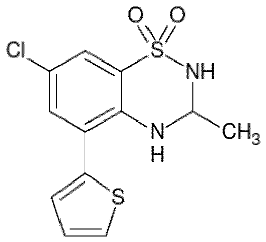

5. The compound of claim 1, wherein R3 is a 5- or 6-membered aromatic ring substituted with at least one substituent group selected from -OY1, -COOY1, - NY1 Y2, where Y1 and Y2 are, independently of each other, H or C₁-C₃.

6. The compound of claim 5, selected from:
8) 3-(7-chloro-3-methyl-1,1-dioxide-3,4-dihydro-2*H*-1,2,4-benzothiadiazin-5-yl) benzoic acid 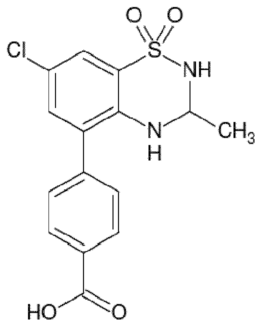
9) 3-(7-chloro-3-methyl-1,1-dioxide-3,4-dihydro-2*H*-1,2,4-benzothiadiazin-5-yl)aniline

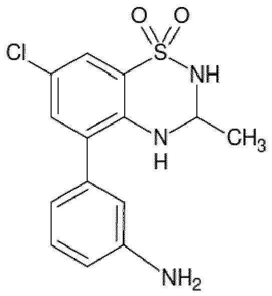

7. Process for the preparation of the compound of formula (I) of claim 1 comprising the steps of:
a) halogenating at position 3 a compound of formula (i) 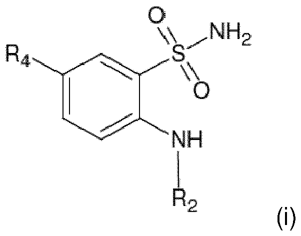
b) condensing by reaction with B(OH)₂R3, and
c) cyclising by reaction with R1COH.

8. Process of claim 7, wherein the step c) is performed before the step b).

9. The compound of formula (I) of any one of claims 1-6 for use as allosteric modulator of AMPA receptor in the treatment of mnemocognitive disorders ascribable to an alteration of the glutamatergic neurotransmission.

10. A pharmaceutical composition comprising at least one compound of any one of claims 1-6 and suitable pharmaceutically acceptable excipients.
